(19) Europäisches Patentamt
European Patent Office
Office européen des brevets

(11) Veröffentlichungsnummer: **0 000 509**
**B1**

(12) **EUROPÄISCHE PATENTSCHRIFT**

(21) Anmeldenummer: **78100378.5**

(22) Anmeldetag: **12.07.78**

(51) Int. Cl.³: **C 07 C 85/12**, C 07 C 93/04, C 07 C 87/123, C 07 C 87/127

(54) **Verfahren zur Herstellung von sekundären aliphatischen Aminen**

(30) Priorität: **25.07.77 CH 9196/77**

(43) Veröffentlichungstag der Anmeldung: **07.02.79 Patentblatt 79/03**

(45) Bekanntmachung des Hinweises auf die Patenterteilung: **06.08.80 Patentblatt 80/16**

(84) Benannte Vertragsstaaten: **BE CH DE FR GB NL SE**

(56) Entgegenhaltungen:
**The Journal of Organic Chemistry, Vol. 34, Nr. 3, März 1969, R. F. Borch "Nitrilium salts—A new method for the synthesis of secondary amines", Seiten 627—629**
**Journal of Economic Entomology, Vol. 65, Nr. 1, Februar 1972, R. E. Cline "Lethal effects of aqueous formulations containing fatty amines or acids against eggs and larvae of Aedes Aegypti", Seiten 177—181**
**Chemical Abstracts, Vol. 76, Nr. 15, 10. April 1972, Columbus, Ohio, USA Ishizuka, Hiroshi et al. "Fundamental studies of anthelmintics 18. Antiparasitic effects of monoamine compounds upon various parasites in vitro"**

(73) Patentinhaber: **Hoechst Aktiengesellschaft**
**Postfach 80 03 20**
**D-6230 Frankfurt**
**Main 80 (DE)**

(72) Erfinder: **Müller, Heinz, Dr.**
**Kantstrasse 43**
**D-8261 Burgkirchen**
**Alz (DE)**
**Becker, Adolf**
**Gerhard-Hauptmann-Strasse 6**
**D-8261 Burgkirchen**
**Alz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von sekundärer aliphatischen Aminen

Sekundäre aliphatische Amine, insbesondere die sogenannten Fettamine mit langen Kohlenstoffketten, werden in großtechnischen Prozessen heute im wesentlichen nach zwei grundlegenden Verfahrensweisen in mannigfaltigen Variationen hergestellt, nämlich durch die Hydrierung von entsprechenden Nitrilen oder durch die sogenannte Aminolyse oder Ammonolyse, d.h. die Umsetzung von Fettalkoholen mit Ammoniak oder primären Aminen in Gegenwart von Wasserstoff.

Die Hydrierung von langkettigen aliphatischen Nitrilen zu entsprechenden sekundären Aminen ist seit langem bekannt. Sie wird großtechnisch bei mittleren bis hohen Drücken von etwa 20 bis 300 bar und bei erhöhten Temperaturen von etwa 130 bis 260°C vorgenommen, wobei sowohl zweistufige als auch einstufige Verfahren entwickelt wurden. Als Hydrierungs-Katalysatoren dienen die verschiedensten Kontakte und Kontaktsysteme auf der Basis von Nickel, Kobalt, Kupferchromit, die gegebenenfalls noch mit anderen Metallen dotiert sein können und häufig in Form von Festbettkontakten eingesetzt werden. Solche Verfahren zur Herstellung sekundärer Amine aus Nitrilen sind beispielsweise bekannt aus der DT—PS 936 518, der DT—PS 1 280 243, der DT—PS 1 941 290, der US—PS 2 781 399, der US—PS 2 784 232 und der GB—PS 836 364.

Daneben gewinnt die Umsetzung von Fettalkoholen mit Ammoniak für technische Prozesse steigende Bedeutung. Ein solches Verfahren ist insbesondere bekannt aus der DT—AS 22 55 701. Danach können bei Temperaturen von 120 bis 250°C langkettige Fettalkohole mit Ammoniak in Gegenwart von Wasserstoff an Hydrier-Dehydrierkatalysatoren bei atmosphä rem Druck in die entsprechenden sekundären Amine überführt werden.

Ferner ist aus der DT—AS 1 219 493 ein Verfahren bekannt, nach dem tertiäre Amine mit 6 bis 26 C—Atomen in jeder aliphatischen Gruppe aus Gemischen der entsprechenden aliphatischen Alkohole mit entsprechenden Nitrilen hergestellt werden, in dem bei Temperaturen zwischen 160 und 280°C und bei einem Druck zwischen 7 und 14 bar in Gegenwart von Hydrierungskatalysatoren durch die in flüssiger Phase vorliegenden Ausgangsstoffe Wasserstoff geleitet wird. Während der Reaktion gebildeter Wasserdampf bzw. gebildetes Ammoniak wird durch den im Überschuß hindurchgeleiteten Wasserstoff aus der Reaktion entfernt. Primäre und sekundäre Amine können unter diesen Bedingungen praktisch nur in unbedeutenden Mengen als Nebenprodukte erhalten werden.

Aus der DT—AS 15 43 317 ist schließlich ein Verfahren bekannt, bei dem Alkohole und Nitrile in Gegenwart von wasserfreiem Fluorwasserstoff zu Amiden umgesetzt und diese Amide anschließend in Gegenwart von Hydrolysemitteln zu Gemischen von Carbonsäuren und Aminen hydrolysiert werden. Für eine großtechnische Herstellung von Fettaminen kommt dieses Verfahren allein schon wegen der umständlichen Isolierung des reinen Amins aus dem Hydrolyse-Gemisch kaum in Betracht.

Sekundäre Amine mit langen C-Ketten besitzen insbesondere bezüglich ihrer Weiterverarbeitung zu den entsprechenden quartären Ammoniumsalzen und deren Einsatz als Weichmacher für Textilien hohes technisches Interesse. Andererseits stößt ihre Herstellung nach den verschiedenen Verfahren stets auf besondere Schwierigkeiten, da es in der Reaktionskette primäres Amin—sekundäres Amin—tertiäres Amin relativ leicht gelingt, die Anfangs- oder Endstufe zu gewinnen, aber wesentlich schwieriger ist, die Zwischenstufe, nämlich das sekundäre Amin, in hohen Ausbeuten zu erhalten. Es bestand jedoch ein Bedürfnis, die technisch interessante Reaktion von Fettalkoholen mit Fettnitrilen auch für die Herstellung sekundärer Fettamine zu nutzen.

Es wurde nun gefunden, daß dies gelingt mit einem Verfahren zur Herstellung von sekundären aliphatischen Amine aus aliphatischen Nitrilen und aliphatischen Alkoholen in flüssiger Phase unter Hindurchleiten von Wasserstoff in Gegenwart von Hydrier-Dehydrierkatalysatoren bei erhöhten Temperaturen und unter Abführung des Reaktionswassers, das dadurch gekennzeichnet ist, daß man bei Temperaturen von 120 bis 260°C unter praktisch drucklosen Bedingungen aliphatische Alkohole der Formel

$$R_1OH,$$

worin $R_1$ einen geradkettigen oder verzweigten, in $\alpha$-Stellung jedoch höchstens einfach verzweigten Alkyl-, Alkenyl- oder mehrfach äthylenisch ungesättigten Kohlenwasserstoffrest mit 8 bis 26 C—Atomen oder einen Alkoxyalkylrest der Formel $R_2(OX)_m$— mit einem Molekulargewicht von mindestens 130, in welcher $R_2$ ein geradketiger oder verzweigter Alkyl-, Alkenyl- oder mehrfach äthylenisch ungesättigter Kohlenwasserstoffrest mit 1 bis 26 C—Atomen, X ein Rest $—CH_2CH_2—$, $—CH(CH_3)—CH_2—$ oder $—CH_2—CH(CH_3)—$ und m eine ganze oder gebrochene Zahl zwischen 1 und 20 sein kann und innerhalb von m auch Kombinationen der Reste X vorliegen können, bedeutet,

oder Gemische solcher Alkohole mit aliphatischen Nitrilen der Formel

$$R_3—CN,$$

worin $R_3$ einen geradkettigen oder verzweigten Alkyl-, Alkenyl- oder mehrfach äthylenisch ungesättigten Kohlenwasserstoffrest mit 8 bis 26 C—Atomen oder den Rest eines Äthernitrils der Formel $R_4—(OX)_n—O(CH2)_p—$ mit einem Molekulargewicht von mindestens 130, in welcher $R_4$ ein geradkettiger oder verzweigter Alkyl-, Alkenyl- oder mehrfach äthylenisch ungesättigter Kohlenwasserstoffrest mit 1 bis 26 C—Atomen, X ein Rest der obengenannten Bedeutung, n eine ganze oder gebrochene Zahl zwischen 1 und 20 oder null und p 1 oder 3 sein kann, wobei innerhalb von n auch Kombinationen der Reste X vorliegen können, bedeutet,

oder mit Gemischen solcher Nitrile in einem Molverhältnis Alkohol:Nitril von 90:10 bis 10:90 mit mindestens 2 Mol Wasserstoff pro Mol Nitril in innigen Kontakt bringt, wobei während der gesamten Reaktionszeit eine Ammoniakkonzentration von 3 bis 75 Vol.-% im Reaktionsgas aufrechterhalten wird.

Als Ausgangsstoffe für das erfindungs gemäße Verfahren sind auf der einen Seite aliphatische Alkohole der Fomel

$$R_1OH$$

zu nennen, in der $R_1$ einen Alkylrest oder einen ein- oder mehrfach äthylenisch ungesättigten Kohlenwasserstoffrest darstellt, der insgesamt 8 bis 26 C—Atome besitzt. Solche Alkohole können in der Kette eine oder mehrere Kettenverzweigungen in Form sekundärer oder tertiärer C—Atome aufweisen, jedoch besitzen sie in der $\alpha$-Stellung zur OH-Gruppe höchstens eine Verzweigung, das heißt es handelt sich um primäre oder sekundäre Alkohole. Nur beispielsweise seien genannt: n-Octyalkohol, 2-Äthylhexanol, Isoctylalkohol, Isononylalkohol, Laurylalkohol, Isotridecylalkohol, Oleylalkohol, Cetylalkohol und Stearylalkohol. Geeignet sind ferner Alkohole oder Gemische von Alkoholen, die bei der Hydrierung von natürlichen Fettsäuren beziehungsweise Fettsäureestern entstehen, wie zum Beispiel Talgfettalkohol oder Palmkernfettalkohole. Hierzu gehören auch die einfach bis mehrfach ungesättigten Alkohole, die bei der Hydrierung der mehrfach ungesättigten Fettsäure-ester der Tran- und Rapsölfettsäuren entstehen. Genannt seien ferner auch die wohlfeilen primären Alkohole mit bis zu 24 C—Atomen, erhalten durch den Äthylenaufbau nach dem Ziegler-Prozeß. Auch mehr oder weniger verzweigte Alkohole, wie sie durch Oxo synthesen aus geradkettigen oder verzweigten, mittel- oder endständigen ungesättigten Olefinen hergestellt werden, oder der aus Isononylaldehyd zugängliche Isooctadecylalkohol können als Ausgangsalkohole Verwendung finden. Als sekundäre Alkohole kommen beispielsweise solche in Frage, die nach den bekannten Verfahren der Direktoxidation von geradkettigen und verzweigten Paraffinen in Gegenwart von Borsäure zugänglich sind. Die Ausgangsalkohole können in Form beliebiger Gemische vorliegen. Bevorzugt sind geradkettige und verzweigte primäre Alkohole, die gesättigt oder ungesättigt mit 1 bis 2 äthylenischen Doppelbindungen sein können und die 14 bis 22, insbesondere 16 bis 18 C—Atomen aufweisen.

Als Ausgangsalkohole im erfindungsgemäßen Verfahren eignen sich auch Ätheralkohole, wie sie durch Oxäthylierung und/oder Oxpropylierung von primären und sekundären Alkoholen entstehen. In diesen Ätheralkoholen nimmt der Rest $R_1$ die Bedeutung $R_2(OX)_m—$ an. Dabei können die Einheiten ausschließlich von Äthylenoxid oder Propylenoxid abgeleitete Einheiten sein, oder es können auch Gemische solcher Einheiten sein, und zwar in statistischer Verteilung oder in Form von Blöcken. m bedeutet den durchschnittlichen Oxalkylierungsgrad. Daher kann m sowohl eine ganze als auch eine gebrochene Zahl sein, die für die Ausgangsalkohol-Komponente des erfindungsgemäßen Verfahrens zwischen 1 und 20, vorzugsweise zwischen 1 und 8 und insbesondere zwischen 1 und 3 liegt. $R_2$ kann ein geradkettiger oder verzweigter Alkyl-, Alkenyl- oder mehrfach äthylenisch ungesättigter Kohlenwasserstoffrest mit 1 bis 26, vorzugsweise 8 bis 22 C—Atomen sein, jedoch mit der Maßgabe, daß das Molekulargewicht des gesamten Rests $R_2(OX)_m—$ mindestens 130 betragen soll, da nur solche Reste unter den Reaktionsbedingungen des erfindungsgemässen Verfahrens einen genügend niedrigen Dampfdruck des Alkohols bewirken, der verhindert, dass der Ausgangsalkohol mit dem Reaktionswasser aus dem Prozess ausgetragen wird. Die genannten Ätheralkohole können auch im Gemisch mit den obengenannten geradkettigen oder verzweigten, gesättigten oder ungesättigten, primären oder sekundären Alkoholen eingesetzt werden.

Die zweite Reaktionskomponente im erfindungsgemässen Verfahren sind Nitrile der Formel

$$R_3CN,$$

in denen der Rest $R_3$ gleichfalls einen Alkylrest oder einen ein- oder mehrfach äthylenisch ungesättigten Kohlenwasserstoffrest darstellen kann, wobei hier jedoch zum Unterschied von den Ausgangsalkoholen auch in $\alpha$-Stellung ein tertiäres C—Atom stehen kann. Im übrigen gilt bezüglich der Kettenverzweigungen in $R_3$ das gleiche, was für die Reste $R_1$ der Ausgangsalkohole gesagt wurde. Diese Reste $R_3$ weisen 8 bis 26 C—Atome auf. Bevorzugt sind geradkettige und verzweigte gesättigte Nitrile oder solche mit bis zu 3 äthylenischen Doppelbindungen, die 14 bis 22, insbesondere 16 bis 18 C—Atome aufweisen. Auch die Ausgangsnitrile können in Form beliebiger Gemische in

den genannten Grenzen eingesetzt werden.

Die Herstellung der als Ausgangskomponenten im erfindungsgemässen Verfahren benötigten Nitrile erfolgt nach bekannten Verfahren aus den gleichkettigen Carbonsäuren durch Umsetzung mit Ammoniak unter dehydratisierenden Bedingungen. Ferner sind solche Nitrile zugänglich durch die sogenannte Ammonoxidation von Kohlenwasserstoffen oder durch die Nitrilierung von Alkoholen mit Ammoniak, die beforzugt an Eisenkontakten erfolgt.

Als Ausgangsnitrile sind auch Äthernitrile geeignet, wobei der Rest $R_3$ dann die Bedeutung $R_4$—$(OX)_n$—$O(CH_2)_p$— annimmt, worin $R_4$ ein geradkettiger oder verzweigter Alkyl-, Alkenyl- oder mehrfach äthylenisch ungesättigter Kohlenwasserstoffrest mit 1 bis 26 C—Atomen, vorzugsweise mit 8 bis 22 C—Atomen sein kann. (OX) sind auch hier Einheiten, die durch Oxalkylierung mit Äthylenoxid und/oder Propylenoxid entstehen und die wiederum, wenn beide Oxalkylatreste vertreten sind, statistisch verteilt oder in der Art von Blockcopolymeren vorliegen können. Der Oxalkylierungsgrad n kann die gleichen Werte annehmen wie m in den Ausgangsätheralkoholen, er kann bei den Äthernitrilen jedoch auch null werden. Der Index p schliesslich kann 1 oder 3 sein. Auch für die Äthernitrile gilt die Massgabe, dass der gesamte Rest $R_4$—$(OX)_n$—$O(CH_2)_p$— mindestens das Molekulargewicht 130 aufweisen soll. Die genannten Äthernitrile sind nach bekannten Verfahren darstellbar, beispielsweise diejenigen, in denen $p = 1$, durch Umsetzung von Chlormethyläthern mit Kupfercyanid, wobei die entsprechenden Chlormethyläther wiederum durch Umsetzung von Alkoholen oder oxalkylierten Alkoholen mit Formaldehyd und HCl gewonnen werden können. Äthernitrile, in denen der Index $p = 3$ ist, kann man gewinnen über die entsprechenden Ätheralkohole, die wiederum durch Umsetzung von Alkoholen oder oxalkylierten Alkoholen mit Oxetan (1,3-Propylenoxid) darstellbar sind.

Auch die in den vorstehend genannten Grenzen einsetzbaren Nitrile und/oder Äthernitrile können in Form von Gemischen vorliegen.

Die Ausgangsalkohole und die Ausgangsnitrile liegen während der Umsetzung in flüssiger Phase vor.

Die Umsetzung im Rahmen des erfindungsgemässen Verfahrens erfolgt unter drucklosen Bedingungen, das heisst praktisch unter Normaldruck. Geringe Drucherhöhungen auf etwa 0,5 bis 1 bar Überdruck, die sich beispielsweise aus Widerständen der Leitungen und auch aus der Überwindung der Flüssigkeitshöhe als Folge des Einleitens der Gase ergeben, werden hier dem praktisch drucklosen Bereich ebenso zugerechnet wie ein geringer Unterdruck, der sich beispielsweise durch eine kleine Druckdifferenz der Gasumwälzpumpe auf der Saugseite der Apparatur ergeben kann.

Die Temperatur während der Umsetzung nach dem erfindungsgemässen Verfahren soll zwischen etwa 120 und etwa 260°C liegen, bevorzugt wird zur Erzielung wirtschaftlicher Reaktionsgeschwindigkeiten in einem Temperaturbereich zwischen etwa 180 und etwa 260°C gearbeitet. Jedoch findet auch im letzteren Bereich bei den darunter liegenden Temperaturen ab 120°C, die gegebenenfalls bewusst langsam durchlaufen werden sollen, bereits eine merkliche Umsetzung zu den gewünschten sekundären Aminen statt.

Als Katalysatoren werden für das erfindungsgemässe Verfahren solche Kontakte benötigt, die gleichzeitig eine Hydrier- und eine Dehydrierfunktion besitzen. Dies sind bevorzugt Nichelkontakte, die sowohl in Form der aktiven Raney-Nickel-Typen als auch in Korn- oder Pulverform mit oder ohne Trägermaterial vorliegen können. Geeignet sind ferner die entsprechenden Kobaltkontakte oder Nickel-Kobalt- oder Nickel-Kobalt-Kupfer-Mischkontakte, ferner auch Kupferchromit-Katalysatoren, die gegebenenfalls mit Zuschlägen von Kupferoxid, Alkali- oder Erdalkalimetallen, wie insbesondere Barium, versehen sein können. Besonders geeignet für den erfindungsgemässen Prozess sind die Nickel- und Kobalt-Katalysatoren der verschiedensten Arten mit und ohne Zuschläge anderer Metalle sowie mit Trägern und Aktivatoren. Als Zuschläge und Träger seien genannt Mangan, Eisenoxid, Zinkoxid, Aluminiumsilikate, Aluminiumoxid sowie $SiO_2$ in Form von Kieselgur oder als geblasenes Syntheseprodukt. Die Nickelkontakte verdienen den Vorrang.

Wenig geeignet sind reine Edelmetallkontakte auf der Basis von Palladium und Platin, die jedoch brauchbar gemacht werden können, indem sie mit Dehydrierkontakten, wie z.B. Raney-Kupfer oder Kupfer-Chrom-Katalysatoren kombiniert werden. Nach solchen Mischverfahren ist es grundsätzlich möglich, zwei verschiedene handelsübliche Kontakte so abzumischen, dass die gewünschten Hydrier-Dehydrier-Eigenschaften erhalten werden.

Der bevorzugte Temperaturbereich des erfingungsgemässen Verfahrens hängt in gewissen Masse von der Art des Katalysators ab. Beim Einsatz von Nickelkatalysatoren hat sich der Temperaturbereich von 180 bis 200°C als besonders günstig erwiesen. Diese Temperaturen sind insbesondere bei hochsiedenden Ausgangsalkoholen und Ausgangsnitrilen ab etwa 12 C—Atomen gut einstellbar. Bei Reaktanten mit kürzeren Ketten kann die optimale Temperatur ab etwa 180°C nicht sofort eingestellt werden. Man beginnt in diesem Fall die Umsetzung bei 120°C und leitet zunächst eine relativ geringe Menge des Reaktionsgases in das Nitril-Alkohol-Gemisch ein. Bei dieser Temperatur beginnt bereits die Umsetzung in hochsiedende sekundäre Amine oder in deren Vorprodukte. Mit fortschreitender Reaktion kann dann sowohl die Temperatur als auch die Gasmenge langsam angehoben werden.

Verwendet man anstelle von Nickelkontakten die etwas trägeren Kupfer-Chrom-Katalysatoren, so liegt die optimale Reaktionstemperatur bei etwa 230 bis 250°C. In diesem Fall können Nitril-Alkohol-Gemische ab etwa 15 bis 16 C—Atomen oder solche etwa gleicher Siedepunkte beziehungsweise entsprechender Dampfdrücke unmittelbar auf die Reaktionstemperatur eingestellt werden, ohne dass hier eine langsame Aufheizperiode durchlaufen werden muss.

Die Menge an Katalysator für das erfindungsgemässe Verfahren ist ausser von Typ des Kontaktes unter anderem von der gewählten Reaktionstemperatur abhängig. Sie bewegt sich innerhalb des erfindungsgemässen Verfahrens in Grenzen zwischen 1 und 6 Gew.-%, bezogen auf die Gewichtsmenge der Reaktanten und berechnet auf aktives Metall oder Metalloxid. Beim Einsatz von beispielsweise annähernd äquimolaren Mischungen von Alkoholen mit gemischten $C_{16}/C_{18}$- Nitrilen betrug die erforderliche Kontaktmenge an Raney-Nickel unter sonst gleichen Bedingungen bei 180°C 5 Gew.-%, bei 200°C 2 Gew.-% und bei 210°C 1 Gew.-%. Bei 210°C machten sich in geringem Mass Nebenreaktionen bemerkbar. Die genannten Zahlen gelten für den einmaligen Einsatz eines frischen Kontakts.

Während der Umsetzung sind dem Reaktionsgemisch pro Mol Nitril mindestens zwei Mol Wasserstoff anzubieten, ein Überschuss von Wasserstoff, der beliebig gross sein kann, stört nicht. Dabei ist insbesondere auch die Menge an Wasserstoff zu berücksichtigen, die gegebenenfalls für die Hydrierung von äthylenisch ungesättigten Doppelbindungen der Ausgangsnitrile oder -alkohole erforderlich ist. Wasserstoff muss dem Reaktionsgemisch von Anfang an angeboten werden, da andernfalls unerwünschte Nebenreaktionen auftreten.

Die im Reaktionsgas vorhandene Wasserstoffkonzentration errechnet sich aus der weiter unten genannten Ammoniakkonzentration als Differenz gegen 100%. Als Reaktionsgas wird hier und im folgenden nur die Summe von Wasserstoff und Ammoniak verstanden.

Entscheidend für die Umsetzung von Alkoholen und Nitrilen zu sekundären Aminen nach dem erfindungsgemässen Verfahren ist es, dass während der gesamten Reaktionszeit neben Wasserstoff auch Ammoniak im Reaktionsgas vorhanden ist. Es muss daher dafür Sorge getragen werden, dass der Ammoniak nicht unkontrolliert entweicht, da ein Ammoniakdefizit die Ausbeute an sekundärem Amin entscheidend verschlechtert. Je nach dem angewandten Molverhältnis der Reaktanten kann die Ammoniakkonzentration zwischen 3 und 75 Vol.-% im Reaktionsgas liegen. Sie kann sowohl innerhalb dieses Bereiches während der gesamten Reaktionszeit konstant gehalten werden als auch innerhalb dieser Grenze schwanken. Dabei können die genannten Grenzen für kurze Zeit insbesondere überschritten, gegebenenfalls auch geringfügig unterschritten werden. Entscheidend ist jedoch, dass die Ammoniakkonzentration so kontrolliert wird, dass sie im wesentlichen während der Reaktionsdauer innerhalb dieser Grenzen aufrechterhalten wird. Wird die Grenze der Ammoniakkonzentration im Reaktionsgas unterschritten, so muss für eine Erhöhung, wird sie überschritten, so muss für eine Absenkung der Ammoniakkonzentration gesorgt werden. Dies gilt sowohl für Ammoniakanteile, die von aussen zu- oder abgeführt werden, als auch für solche, die aus den Reaktionspartnern selbst entstehen.

Der Anteil der Reaktionspartner Alkohol und Nitril kann im erfindungsgemässen Verfahren in weiten Grenzen, bis etwa zu einem Molverhältnis Alkohol : Nitril zwischen 90 : 10 und 10 : 90, schwanken. Bevorzugt ist ein Molverhältnis Alkohol : Nitril zwischen 70 : 30 und 30:70 und insbesondere die Reaktion zwischen äquimolaren Anteilen beider Partner, wobei unter äquimolar bezüglich der technischen Gegebenheiten (beispielsweise der Reinheit und der Einheitlichkeit der Ausgangsstoffe) hier ein Molverhältnis Alkohol : Nitril zwischen 60 : 40 und 40 : 60 verstanden werden soll. Für diesen letzteren Fall soll sich die Ammoniakkonzentration etwa zwischen 3 und 60 Vol.-% und vorzugsweise zwischen 3 und 50 Vol.-% bewegen. Vorteilhafterweise gilt, dass sich die Ammoniakkonzentration bei einem grossen Überschuss an Nitril mehr im unteren Teil des Bereiches, bei grossem Überschuss an Alkohol mehr im oberen Teil des Bereiches halten soll. Im letzteren Fall besteht ferner die Massgabe, dass insgesamt pro Mol an überschüssigem Alkohol mindestens eine Menge von 1/2 Mol Ammoniak anzubieten ist.

Unter den Bedingungen des erfindungsgemässen Verfahrens und in Abhängigkeit von den Molverhältnissen der eingesetzten Ausgangskomponenten Nitril und Alkohol soll sich die Gasgeschwindigkeit, mit der das Reaktionsgas, gegebenenfalls einschliesslich Inertgas, die flüssige Phase passiert, beziehungsweise mit ihr in innigen Kontakt kommt, zwischen 200 und 600 l Gas pro kg Reaktantengemisch und Stunde bewegen Diese Grenzen sind nicht absolut kritisch, jedoch verläuft die Umsetzung bei niedrigeren Gasgeschwindigkeiten, etwa 50 bis 100 l pro kg und Stunde, langsamer. Auf der anderen Seite bringen Gasmengen oberhalb von 600 l pro kg und Stunde nur dann Vorteile, wenn dafür gesorgt wird, dass sich die grösseren Mengen in der flüssigen Phase auch optimal fein verteilen können. Über 1000 l pro kg und Stunde hinaus sind der Gasmenge vorwiegend Grenzen von Seiten der technischen Durchführbarkeit und der Wirtschaftlichkeit gesetzt. Der für die Reaktion erforderliche innige Kontakt zwischen Flüssigkeit, Gas und Katalysator wird üblicherweise dadurch hergestellt, dass das Gas unter sehr gutem Rühren oder Umpumpen der flüssigen Phase mit dem suspendierten Kontakt

direkt eingeleitet oder umgewälzt wird. Besonders vorteilhaft sind für eine solche Umwälzung Strahlreaktoren. Bei diesen wird das flüssige Reaktionsgut in einer Strahldüse innigst mit dem Katalysator und den reaktiven Gasen vermischt, wodurch eine besonders schnelle Reaktion erfolgt. Die Strahldüse sorgt gleichzeitig für den Umlauf der Gasphase und die Abscheidung des Reaktionswassers ausserhalb des Kessels. Eine möglichst hohe Gasmenge ist auch für die schnelle und problemlose Austragung des gebildeten Reaktionswassers von grosser Bedeutung. Zur Beschleunigung der Austragung des Reaktionswassers und aus Gründen einer Kostenersparnis kann das Reaktionsgas, das heisst das Gemisch aus Wasserstoff und Ammoniak, mit Anteilen von Inertgasen wie Stickstoff oder Methan, verdünnt sein. Inertgase können in Anteilen von 0 bis 50 Vol.-% im Gasgemisch zusätzlich zu den reaktiven Gasen Wasserstoff und Ammoniak anwesend sein. Derartige Gase reduzieren zwar die Partialdrücke von Wasserstoff und Ammoniak, aber andererseits fördern sie den wichtigen Austrag des Reaktionswassers.

Die Reaktion kann sowohl nach der sogenannten offenen wie auch der sogenannten geschlossenen Fahrweise durchgeführt werden. Die offene Fahrweise besteht darin, dass man unter gutem Rühren durch das mit dem Reaktionsgemisch aus Alkohol und Nitril beschickte Reaktionsgefäss, das gleichzeitig den Katalysator enthält, bei der erforderlichen Reaktionstemperatur Wasserstoff hindurchleitet, wobei erforderlichenfalls der für die Aufrechterhaltung der Ammoniakkonzentration im Reaktionsgemisch notwendige Anteil an Ammoniak mit zugeführt wird. Der Ausgang der Apparatur ist über einen absteigenden Kühler, der vorteilhafterweise eine Vorlage für die Aufnahme des Reaktionswassers enthält, offen mit der Atmosphäre verbunden. Die überschüssigen Reaktionsgase verlassen die Apparatur über eine Abgasleitung.

Die geschlossene Fahrweise, die auch als Kreisgasfahrweise bezeichnet werden kann, ist die bevorzugte Aufführungsform des erfindungsgemässen Verfahrens. Sie unterscheidet sich apparativ von der sogenannten offenen Fahrweise dadurch, dass Wasserstoff und Ammoniak nach Auskondensation des Reaktionswassers mittels eines gut wirksamen Kondensators durch eine Umwälzpumpe im Kreis geführt werden. Der verbrauchte Wasserstoff und Ammoniak werden in die Apparatur nachgespeist. Man kommt bei der geschlossenen Fahrweise daher bei gleich hohen Ausbeuten an sekundären Aminen mit wesentlich geringeren Mengen der beiden Gase aus. Der Ammoniakverlust kann noch dadurch besonders niedrig gehalten werden, wenn vorteilhafterweise das abgeschiedene Reaktionswasser auf etwa 90°C eingestellt wird und die abziehenden Gase über einen Rückflusskühler kondensieren können. Statt eines etwa 10 gew.-%igen Ammoniakwassers fällt bei dieser Massnahme eine 1 bis 3 ge.-%ige Ammoniaklösung an.

Bei der Kreisgasfahrweise kann es sich als günstig erweisen, ein- oder mehrmals, das heisst chargenweise, während des Prozesses oder auch kontinuierlich eine bestimmte Gasmenge aus dem Kreislauf auszufahren und als Ersatz frisches Gas einzufahren. Hierdurch kann die Anreicherung unerwünschter Gase, wie sie sich in kleinen Mengen durch Nebenreaktionen bilden, verhindert werden. Die Aus- und Einfuhr der Gase kann sowohl zur Regulierung der Ammoniak-, Wasserstoffals auch der Inertgaskonzentration genutzt werden. Die Apparatur für die Kreisgasfahrweise des erfindungsgemässen Verfahrens besitzt ausser dem Kondensator auch eine einschaltbare Vorrichtung, mit deren Hilfe überschüssiger Ammoniak, der sich im Kreisgas befindet, ganz oder teilweise entfernt werden kann.

Die Vorrichtung zur Ammoniakregulierung befindet sich vorteilhafterweise im Nebenschluss des Gaskreislaufes der Apparatur. Sie tritt in Funktion, sobald die gewünschte Ammoniakkonzentration überschritten wird. Die genannte Vorrichtung kann beispielsweise ein mit flüssigem Absorptionsmittel gefüllter Waschturm oder Rührbehälter, aber auch ein mit festen Adsorbentien gefüllter Adsorptionsturm sein.

Als Adsorbens für Ammoniak in der genannten Vorrichtung kann beispielsweise Wasser oder wässrige Schwefelsäure dienen. Auch konzentrierte Schwefelsäure ist verwendbar, die im Bedarfsfall in einer bestimmten Stelle des Gasumlaufs dosiert eingespritzt wird.

Die Überwachung der Ammoniakkonzentration im Reaktionsgas erfolgt beispielsweise mit Hilfe eines Infrarotanalysengerätes, eines Prozesschromatographen oder auch mit Hilfe eines sonstigen kontinuierlichen Analysengeräts, das nach chemischen oder physikalischen Methoden Analysenwerte liefert. Das eingesetzte Analysengerät kann vorteilhafterweise die Zuführung und Abführung von Ammoniak automatisch regeln.

Nach Absenkung der Ammoniakkonzentration durch die genannten Massnahmen ist es erforderlich, die abgeführte Gasmenge durch Wasserstoff, gegebenenfalls im Gemisch mit Inertgasen, im Kreislauf zu ersetzen.

Als besonders günstige Massnahme kann die Ammoniakkonzentration durch ein Kühlaggregat eingestellt werden, das sowohl Ammoniak dem Kreislauf entnehmen, als auch Ammoniak an ihn abgeben kann. Ein solches Aggregat kann sowohl im Hauptkreislauf als auch im Nebenkreislauf installiert sein.

Bei der sogenannten offenen Fahrweise erübrigt sich eine Vorrichtung zur Ammoniakregulierung, weil hier die Gase direkt und in der richtigen Zusammensetzung in die Apparatur eingeleitet werden. Sowohl bei der offenen Fahrweise als auch bei der Kreisgasfahrweise ist es wichtig, dass das entstehende Wasser

schnell aus der flüssigen Reaktionsphase entfernt wird und die auskondensierte Menge in einer Vorlage gemessen werden kann. Die abgeschiedene Wassermenge und die Aufnahme des Wasserstoffs sind ein Mass für den Reaktionsablauf. Die Beendigung der Reaktion nach dem erfindungsgemässen Verfahren kann mit Hilfe der titrierbaren Alkalität des gewünschten sekundären Amins festgestellt werden.

Die Schilderung der Verfahrensweisen erfolgte unter dem Gesichtspunkt eines chargenweisen Verfahrens. Es ist aberebenso möglich, das erfindungsgemässe Verfahren ganz oder teilweise kontinuierlich durchzuführen, beispielsweise in einem Rohrsystem, in mehreren hintereinandergeschalteten Reaktoren oder in kaskadenartig angeordneten Reaktionskesseln.

Innerhalb des erfindungsgemässen Verfahrens können auch ungesättigte Nitrile und Alkohole, wie oben bereits erwähnt, eingesetzt werden. Die ungesättigten Alkylketten können sowohl eine als auch mehrere Doppelbindungen enthalten. Es seien beispielsweise genannt: Oleylalkohole mit Jodzahlen von 50 bis 95, Talgfettnitril mit einer Jodzahl von etwa 50 sowie die Nitrile von Tranöl- und Rapsölfettsäuren mit Jodzahlen grösser als 100, ferner Oleylnitril und Sojaölnitril. Dabei kann das Verfahren so gelenkt werden, dass sowohl gesättigte als auch die entsprechenden ungesättigten oder partiell ungesättigte sekundäre Amine erhalten werden. Zur Erhaltung der Doppelbindungen eignen sich am besten Kupfer-Chrom-Katalysatoren. Ungesättigte oder partiell ungesättigte sekundäre Amine können auch aus ungesättigten Nitrilen und Alkoholen mit Nickelkontakten erhalten werden. In diesem Fall ist ein Reaktionsgas besonders vorteilhaft, das neben Wasserstoff etwa 10 bis 60, vorzugsweise 20 bis 50 Vol.-% Ammoniak enthält.

Zu gesättigten sekundären Aminen aus ungesättigten Ausgangskomponenten gelangt man am vorteilhaftesten, wenn sich der Ammoniakgehalt des Reaktionsgases im unteren Bereich der angegebenen Grenzen bewegt und wenn man nach Beendigung der Bidung des sekundären Amins das Reaktionsgas durch reinen Wasserstoff ersetzt. Gegebenenfalls kann dabei die Temperatur erhöht und die Gasmenge vergrössert werden.

Das erfindungsgemässe Verfahren der Bildung des sekundären Amins dauert üblicherweise 1,5 bis 4, vorzugsweise 2 bis 3 Stunden. Für die völlige Hydrierung vorhandener Doppelbindungen wird im Extremfall (bei hoher Jodzahl) nochmals in etwa die gleiche Zeit benötigt. Eine Verkürzung der Nachhydrierungsphase kann erreicht werden, wenn man bei erhöhtem Druck, etwa bei 4 bis 10 bar, arbeitet.

Die nach dem erfindungsgemässen Verfahren erhaltene Ausbeute an Aminen, berechnet als Gewichtsprozente, liegt bei etwa 90 bis 99%, in den meisten Fällen beträgt sie mehr als 95%, ist also nahezu quantitativ. Die Differenz besteht aus nicht-aminischen Bestandteilen, insbesondere kleinen Mengen von Abbauprodukten der Ausgangsalkohole und -nitrile sowie Verunreinigungen bei Ausgangsmaterialien von technischer Qualität. Der Anteil an den gewünschten sekundären Aminen liegt bei 80 bis 95 Mol-%, in den meisten Fällen über 90 Mol-%, bezogen auf die Gesamtausbeute an Aminen gleich 100 Mol %. Der Rest sind primäre und tertiäre Amine, wobei die primären Amine häufig völlig abwesend sind. Die Farbqualität der erhaltenen sekundären Amine ist sehr gut.

Es werden mit Sicherheit Jodfarbzahlen von 0,2 bis 2 Einheiten erhalten (Jodfarbzahlen nach DIN-Norm 6162). Die erhaltenen sekundären Amine bedürfen im allgemeinen für die Weiterverarbeitung keines besonderen Reinigungsprozesses durch ein Destillations- oder Absorptions-Verfahren.

Es war bekannt, dass die Hydrierung von Nitrilen zu sekundären Aminen nur unter mittleren bis hohen Drücken befriedigend durchgeführt werden kann. Daher ist es als überraschend anzusehen, dass die Umsetzung von Alkoholen und Nitrilen in wechselnden Molverhältnissen drucklos durchgeführt werden kann und dennoch hohe Ausbeuten an den gewünschten sekundären Aminen liefert. Es kann jedoch beim erfindungsgemässen Verfahren auch geringer Überdruck angewendet werden.

Die nach dem erfindungsgemäßen Verfahren herstellbaren sekundären Amine sind vor allem wertvolle Zwischenprodukte. Sie werden bevorzugt zur Herstellung von Weichmachern für Textilien, von Komponenten für organophile Ammoniumbentonite sowie von Mikrobiociden, speziell zur Bakterien-, Pilzund Algen-Bekämpfung, verwendet. Ferner dienen sie zur Herstellung von Antistatika, Konditionierungs- und Präparationshilfsmitteln, für die Haarkosmetik und für Synthesefasern. Sekundäre Amine mit einer Gesamtzahl von mehr als 20 C—Atomen können auch zur Flüssig-Extraktion von Metallen, wie beispielsweise Wolfram, in stark sauren Lösungen eingesetzt werden.

Die nach dem erfindungsgemäßen Verfahren herstellbaren sekundären Amine kann man im Hinblick auf die beiden am Stickstoffatom gebundenen Reste in symmetrische sekundäre Amine (beide Reste bestehen aus einem reinen Kohlenwasserstoffrest oder aus einem Äthoxy- und/oder Propoxygruppen enthaltenden Kohlenwasserstoffrest) und unsymmetrische sekundäre Amine (ein Rest besteht aus einem reinen Kohlenwasserstoffrest und der andere aus einem Äthoxy- und/oder Propoxygruppen enthaltenden Kohlenwasserstoffrest) einteilen.

Im folgenden wird die Erfindung durch Beispiele erläutert:

Beispiel 1

Die Apparatur besteht aus einem Reaktionskolben, der mit einer Gaseinleitung, mit Rührer,

Kontaktthermometer und mit einer bis 90°C heizbaren Raschig-Kolonne versehen ist. Auf dieser Kolonne ist ein Wasserabscheider installiert, der im Bedarfsfall ebenfalls bis 90°C aufgeheizt werden kann, wenn die Konzentration des gelösten Ammoniaks im abgeschiedenen Reaktionswasser möglichst niedrig gehalten werden soll. Auf dem Wasserabscheider befindet sich ein guter Intensivkühler. Daran schliesst sich ein Absorptionsgefäss mit 1-normaler Schwefelsäure an, in dem ausgeschleuster Ammoniak titrimetrisch erfasst werden kann. Das Reaktionsgas in der Apparatur wird durch ein Umwälzpumpe im Kreis geführt. Für die Reaktion benötigter Wasserstoff wird so in den Kreislauf eingeschleust, dass in der Apparatur ein Überdruck bis zu 0,1 bar herrschen kann. Am ausgang der Apparatur kann Abgas abgenommen werden. Das $H_2SO_4$—Absorptionsgefäss lässt sich so schalten, dass das Kreisgas sowohl voll als auch im Nebenschluss des Kreislaufs darüber geführt werden kann. Auch ist es möglich, die Absorption des Ammoniaks ausserhalb des Kreisgases im Abgas in einem $H_2SO_4$—Absorptionsgefäss vorzunehmen.

In die eben beschriebene Apparatur werden 280,0 g = 1,00 Mol Stearylnitril (N-Gehalt 5 Gew.-% nach Kjeldahl) und 1,86,6 g = 0,71 Mol Stearylalkohol (OH—Zahl = 214) vorgelegt. Dies entspricht einem Anteil 58,5 Mol-% an Nitril im Reaktionsgemisch. Man gibt dann 23,3 g = 5 Gew.-% (bezogen auf die Mischung der Reaktanten) eines Nickelkatalysators auf einen Träger mit 55 Gew.-% Nickel hinzu und spült die Apparatur mit Stickstoff. Nach Verdrängung des Stickstoffs durch Wasserstoff wird der Ausgang der Apparatur geschlossen und die Kreislaufpumpe eingeschaltet. Es werden 300 l Kreisgas/kg. Stunde gefahren. Nach Aufheizen auf 200°C wird der Ansatz bei dieser Temperatur über 3 Stunden gefahren, und während dieser Zeit werden 52,6 Wasserstoff zugeführt. Durch Abnahme von 2 l Abfas wird die Ammoniakkonzentration im Kreisgas im Bereich zwischen 3 und 25 Vol.-% gehalten. Nach Beendigung der Reaktion lässt man das Produkt im Kreisgasstrom auf 100°C abkühlen und spült die Apparatur mit Stickstoff. In der Schwefelsäure-Vorlage in der Abgasleitung werden insgesamt 0,131 Mol Ammoniak aufgefangen. Das entstandene Reaktionwasser (16,3 g) enthält weitere 0,023 Mol Ammoniak. Sodann wird der Kolbeninhalt dei ca. 80°C über ein Kieselgurfilter durch eine Nutsche abgesaugt. Es werden 96,3 Gew.-% Gesamtausbeute an Amin mit einer Aminzahl von 17,40 erhalten. Es ist zusammengesetzt aus 92,1 Mol-% an sekundärem Amin, der Rest ist tertiäres Amin, primäres Amin wurde nicht gefunden.

### Beispiel 2

In der in Beispiel 1 beschrieben Kreislaufapparatur werden vorgelegt:

280,0 g = 1,00 Mol Stearylnitril (N-Gehalt 5 Gew.-% nach Kjeldahl)

262,0 g = 1,00 Mol Stearylalkohol (OH—Zahl = 214) und 27,0 g ( = 5 Gew.-%, bezogen auf die Mischung der Reaktanten) eines Nickelkatalysators auf einem Träger (Gehalt 55 Gew.-% Ni). Nach Stickstoffspülung und Verdrängung des Stickstoffs durch Wasserstoff werden, wie in Beispiel 1 beschrieben, 300 l Kreisgas/kg. Stunde in der Apparatur gefahren. Dabei wird auf 200°C aufgeheizt und der Ansatz 2 1/2 Stunden gefahren, wobei während dieser Zeit 50,6 l Wasserstoff und aus einer Kälterfalle 2,78 g = 0,163 Mol Ammoniak in den Kreislauf eingefahren werden, wodurch die Ammoniakkonzentration über die Reaktionszeit im Bereich von 12 bis 27 Vol.-% gehalten wird. Die Aufarbeitung erfolgt wie in Beispiel 1. Es fallen 20,1 g Reaktionswasser an, die 0,034 Mol Ammoniak enthalten. Im Absorptionsgefäss der Abgasleitung werden weitere 0,147 Mol Ammoniak aufgefangen. Es resultiert eine Gesamtamin-Ausbeute von 97,3 Gew.-% mit einer Aminzahl von 17,73. Das Produkt ist frei von primärem Amin, es enthält 96,6 Mol-% sekundäres Amin und 7,4 Mol-% tertiäres Amin.

### Beispiel 3

In der Apparatur gemäss Beispiel 1 werden vorgelegt:

148,3 g = 0,969 Mol Decannitril (entsprechend 74,8 Mol-% Nitril in der Mischung),

51,7 g = 0,327 Mol Decylalkohol sowie

10,0 g eines Träger-Nickelkontaktes (55% Nickel; 5 Gew.-% bezogen auf die Mischung).

Nach Spülen mit Stickstoff und Füllen der Apparatur mit Wasserstoff wird eine Kreisgasmenge von 500 l Kreisgas/kg. Stunde eingestellt und die Apparatur auf 180°C aufgeheizt. Bei dieser Temperatur wird 5 1/2 Stunden gefahren, wobei während dieser Zeit 58 l Wasserstoff eingeschleust werden. Bei Erreichen der Reaktionstemperatur von 180°C enthält das Kreisgas 6 Vol.-% Ammoniak, nach 1/2 Stunde 20 Vol.-% und nach 1 Stunde 34 Vol.-%. Nun wird über den $H_2SO_4$—Absorptionsteil in der Abgasleitung Abgas abgenommen, wodurch das Ammoniakniveau auf 35 Vol.-% gehalten wird. Nach 2 und 3 Stunden Reaktionszeit werden 35 Vol.-% Ammoniak, nach 4 und 5 Stunden 30,5 beziehungsweise 25 Vol.-% gemessen. Am Ende der Reaktionszeit, nach 5 1/2 Stunden, enthält das Kreisgas noch 15 Vol.-% Ammoniak. Bis zu diesem Zeitpunkt werden 13,6 l Abgas kontinuierlich aus der Apparatur abgefahren, die 0,181 Mol Ammoniak enthalten. Ferner fallen 6,9 g Reaktionswasser an, enthaltend weitere 0,061 Mol Ammoniak. Schliesslich werden beim Spülen der Apparatur weitere 0,029 Mol $NH_3$ aufgefangen. Die Gesamtamin-Ausbeute beträgt 95,3 Gew.-% mit einer Aminzahl von 31,85. Darin sind enthalten 3,1 Mol-% primäres Amin, 92,2 Mol-% sekundäres Amin und 4,7 Mol-% tertiäres Amin.

Beispiel 4

In der Kreislaufapparatur, beschrieben in Beispiel 1, werden vorgelegt:
110 g = 0,719 Mol Decannitril (entsprechend 55,8 Mol-% Nitril in der Mischung),
90,0 g = 0,570 Mol Decylalkohol, sowie
10,0 g eines Träger-Nickelkontaktes (5 Gew.-% bezogen auf die Mischung; 55% Nickel).

Die Kreisgasmenge wird auf 500 l/kg. Stunde eingestellt, es wird auf 180°C erwärmt und der Ansatz bei dieser Temperatur 4 1/2 Stunden gefahren, wobei während dieser Zeit 36.4 l Wasserstoff zugeführt werden. Bei Erreichen der Reaktionstemperatur sind im Kreisgas 5 Vol.% Ammoniak, nach 1 Stunde 20 Vol.-% und nach 3 Stunden 30 Vol.-% enthalten. Abgas wird nicht entnommen. Danach fällt die Ammoniakkonzentration bei 3 Stunden auf 18 Vol.%, bei 4 Stunden auf 6 Vol.-%. Am Schluss der Reaktionszeit nach 4 1/2 Stunden werden noch 3 Vol.-% Ammoniak gemessen. Es entstehen 11,3 g Reaktionswasser, die 0,065 Mol Ammoniak enthalten, weitere 0,011 Mol Ammoniak werden beim Spülen der Apparatur aufgefangen. Die Gesamtamin-Ausbeute beträgt 97,4 Gew.-% mit einer Aminzahl von 31,47. Davon sind 0,1 Mol-% primäres Amin, 91,0 Mol-% sekundäres Didecylamin und 8,9 Mol-% tertiäres Tridecylamin.

Beispiel 5

In der Kreislaufapparatur; beschrieben in Beispiel 1, werden vorgelegt:
48,0 g = 0,314 Mol Decannitril,
152,0 g = 0,962 Mol Decylalkohol und
10,0 g eines Nickelkatalysators auf einem Träger (5 Gew.-% bezogen auf die Mischung; 55% Nickel).

Die Kreisgasmenge beträgt 500 l/kg. Stunde, die Reaktionstemperatur 180°C und die Reaktionszeit 4 1/2 Stunden. Während dieser Zeit werden 15,4 l Wasserstoff kontinuierlich zugefahren und gleichzeitig damit aus einer Kältefalle über 3 1/4 Stunden 8,0 g = 0,471 Mol Ammoniak, wodurch sich während der gesamten Reaktionszeit eine Ammoniakkonzentration zwischen 30 und 50 Vol.-% einstellt. Abgas wird nicht entnommen. Im Wasserabscheider werden 19,5 g Reaktionswasser gesammelt, die 0,147 Mol Ammoniak enthalten, weitere 0,048 Mol Ammoniak werden beim Spülen der Apparatur aufgefangen. Das nach Abtrennung des Kontaktes erhaltene Rohprodukt enthält 99,4 Gew.-% Gesamtamin mit einer Aminzahl von 32,05. Darin sind 0,1 Mol-% primäres, 90,3 Mol-% sekundäres und 9,6 Mol-% tertiäres Amin enthalten.

Beispiel 6

In der Kreislaufapparatur, beschrieben in Beispiel 1, werden vorgelegt:
99,5 g = 0,65 Mol Decannitril (entsprechend 50 Mol-% Nitril in der Mischung),
119,6 g = 0,65 Mol eines Octylalkohols, der mit 1 Mol Äthylenoxid oxäthyliert worden ist ($Kp._{10}$ 120 bis 125°C) sowie
10,0 g eines Nickelkatalysators auf einem Träger (5 Gew.-% bezogen auf die Mischung; 55% Nickel).

Die Kreisgasmenge beträgt 500 l/kg. Stunde, die Reaktionstemperatur 180°C und die Reaktionszeit 5 1/2 Stunden. Während dieser Zeit werden 32,9 l Wasserstoff kontinuierlich zugefahren. Nach dem Aufheizen enthält das Kreisgas zu Anfang 7 Vol.-% Ammoniak, nach 1/2 Stunde 25 Vol.-%, nach 1 Stunde 33 Vol.-% und nach 2 Stunden 35 Vol.-% Ammoniak, ohne daß zusätzliche Ammoniakzufuhr erfolgt. Danach sinkt die Ammoniakkonzentration im Kreisgas wieder ab und beträgt am Ende derr Reaktionszeit noch 3 vol.%. Es werden 13,0 g Reaktionswasser erhalten, enthaltend weitere 0,034 Mol Ammoniak. Das nach Abtrennung des Kontaktes erhaltene Rohprodukt enthält 95,8 Gew.% Gesamtamin mit einer Aminzahl von 30,2. Die Bestimmung ergibt, dass darin 84,0 Mol-% sekundäres Amin, 16,0 Mol-% tertiäres Amin und kein primäres Amin enthalten sind, wobei im sek. Amin ($Kp._{13}$ 212—218°C) alle 3 möglichen Spezies gaschromatographisch nachzuweisen sind.

Beispiel 7

In der Kreislaufapparatur, beschrieben in Beispiel 1, werden vorgelegt:
107,0 g = 0,476 Mol Dodecyl-cyanmethyläther (entsprechend 52,4 Mol-% Nitril in der Mischung),
93,0 g = 0,433 Mol Myristylalkohol sowie
5,7 g eines wasserfeuchten Raney-Nickels mit einem Nickelgehalt von 70 Gew.-%, entsprechend 2 Gew.-% Nickel bezogen auf die Reaktionsmischung. Das eingesetzte Nitril und der eingesetzte Alkohol besitzen nach der gaschromatographischen Analyse eine Reinheit von etwa 97%.

Die Kreisgasmenge beträgt 500 l/kg. Stunde, die Reaktionstemperatur 190°C und die Reaktionszeit 6 1/2 Stunden. Nachdem die Apparatur nach der Stickstoffspülung mit Wasserstoff gefüllt worden ist, wird zunächst auf 140°C aufgeheizt und dann gasförmiger Ammoniak in einem Anteil von 10% des Kreisgasvolumens eingeschleust. Während der gesamten Reaktionszeit, also ab Erreichen von 190°C, werden insgesamt 24,1 l Wasserstoff eingefahren. Im Kreislauf erfolgt eine rasche Anreicherung von Ammoniak. Unmittelbar nach Erreichen der Reaktionstemperatur beträgt die Ammoniakkonzentration 27 Vol.-%, nach 1/2 Stunde Reaktionszeit 60 Vol.-% und nach 1 Stunde 67 Vol.-%. Danach sinkt die Ammoniakkonzentration im Kreisgas wieder ab, nach 1 1/2 Stunden auf Vol.-%, nach 2 Stunden auf 19 Vol.% und zum Ende der Reaktionszeit auf 2,9 Vol.-%. Es wird kein Abgas ausgeschleust. In dem auf 90°C beheizten Wasserabscheider werden 8,2 g Reaktionswasser, enthaltend 0,013 Mol $NH_3$, gesammelt. Weitere 0,012 Mol

$NH_3$ werden beim abschliessenden Spülen der Apparatur ausgetragen.

Nach Abtrennen des Kontakts erhält man ein Rohprodukt, das 94,5 Gew.-% Gesamtamin mit einer Aminzahl von 21,08 enthält. Dieses besteht zu 0,4 Mol-% aus primärem, zu 88,3 Mol-% aus sekundärem und zu 11,3 Mol-% aus tertiärem Amin, wobei in sekundären Aminen alle 3 möglichen Spezies gaschromatographisch nachzuweisen sind.

Beispiel 8

In der Kreislaufapparatur, deschrieben in Beispiel 1, werden vorgelegt:
105,0 g = 0,394 Mol Talgfettnitril (Jodzahl 57, N-Gehalt 5,25% nach Kjeldahl),
95,0 g = 0,362 Mol vollständig hydrierter Talgfettalkohol (OH—Zahl 214), sowie
5,7 g wasserfeuchtes Raney-Nickel mit einem Nickelgehalt von 70 Gew.-%, entsprechend 2 Gew.-% Nickel, bezogen auf die Reaktionsmischung.

Die Kreisgasmenge beträgt 500 1/kg. Stunde. Nach dem Füllen der Apparatur mit Wasserstoff wird unter kräftigem Rühren auf 200°C aufgeheizt. Bei einer Temperatur von 140°C wird ein Anteil von 10% des Kreisgasvolumens an gasförmigem Ammoniak eingefahren. Die Reaktionszeit beträgt 2 1/2 Stunden bei einer Temperatur von 200°C, wobei während dieser Zeit 22, 1 l Wasserstoff kontinuierlich in den Kreislauf eingespeist werden. Die Ammoniakkonzentration beträgt zu Beginn der Reaktion 42 Vol.-%, sie fällt nach 1/2 Stunde auf 33 Vol.-%, nach 1 Stunde auf 18 Vol.-% und am Ende der Reaktion auf 11 Vol.-%. In dem auf 90°C beheizten Wasserabscheider sammeln sich während dieser Zeit 7,2 g Wasser, die 0,005 Mol Ammoniak enthalten. Nach Beendigung dieser Reaktion resultiert ein Rohprodukt, das 98 Gew.-% Gesamtamin mit einer Jodzahl von 20 und einer Aminzahl von 19,2 enthält, das zu 4,3 Mol-% aus primärem, 93,1 Mol-% aus selundärem und 2,6 Mol-% aus tertiärem Amin besteht. Dieses Produkt wird bei 200°C 3 Stunden lang in einem Kreisgas weiterbehandelt, das vorher durch Hindurchleiten durch verdünnte Schwefelsäure von Ammoniak befreit wird. Während dieser Zeit werden weitere 3,9 l Wasserstoff zugeführt. Nach 1 Stunde fällt die Jodzahl auf 6, nach 2 Stunden auf 2 und nach 3 Stunden auf 1. Das nunmehr erhaltene Amin hat eine Aminzahl von 19,09, es ist frei von primärem Amin und enthält 95,3 Mol-% sekundäres und 4,7 Mol-% tertiäres Amin.

Beispiel 9

In der Kreislaufapparatur, beschrieben in Beispiel 1, werden vorgelegt:
100,0 g = 0,382 Mol Talgfettnitril (Jodzahl 55, Stickstoffgehalt nach Kjeldahl 5,35%)
100,0 g = 0,381 Mol hydrierter Talgfettalkohol (OH—Zahl 214) und
5,7 g eines wasserfeuchten Raney-Nickels, Nickelgehalt 70%, entsprechend 2% bezogen auf die Reaktionsmischung.

Die Kreisgasmenge beträgt 500 l/kg. Stunde, die Reaktionstemperatur 200°C und die Reaktionszeit 5 1/2 Stunden. Während des Aufheizens werden wiederum bei 140°C etwa 10% des Kreisgasvolumens an gasförmigem Ammoniak in den Kreislauf eingeschleust, ferner wird kontinuierlich Wasserstoff zugeführt. Am Reaktionsbeginn sind im Kreisgas 40 Vol.-%, nach 1/2 Stunde 24 Vol.-%, nach 1 Stunde 11 Vol.-% und am Reaktionsende weniger als 4 Vol.-% Ammoniak enthalten. Zu diesem Zeitpunkt liegt ein Gesamtamin mit einer Ausbeute von 96,8 Gew.-% vor, das eine Jodzahl von 15 und eine Aminzahl von 18,93 besitzt. Es ist zu 4,5 Mol-% aus primärem, zu 92,3 Mol-% aus sekundärem und zu 4,5 Mol-% aus tertiärem Amin. Das Kreisgas wird dann noch 3 Stunden bei 200°C weiter gefahren, ohne dass Ammoniak entnommen wird. Dabei wird weiterhin Wasserstoff zugeführt, sodass die Gesamtmenge an zugeführtem Wasserstoff insgesamt 24,7 l beträgt. Die Ammoniakkonzentration nimmt während dieser Zeit wieder zu und liegt nach 2 Stunden bei 7,4 Vol.-%. Die Jodzahl nimmt ab und liegt nach 3 Stunden bei 2. Nach 5 1/2 Stunden Gesamtreaktionszeit resultiert schliesslich ein Gesamtamin mit einer Aminzahl von 18,93, das 1,0 Mol-% primäres, 92,7 Mol-% sekundäres und 6,3 Mol-% tertiäres Amin enthält.

Beispiel 10

In der Kreislaufapparatur, beschrieben in Beispiel 1, werden vorgelegt:
150,0 g = 0,573 Mol Talgfettnitril (Jodzahl 55, N-Gehalt nach Kjeldahl 5,35%),
50,0 g = 0,191 Mol hydrierter Talgfettalkohol (OH—Zahl 214) und
11,7 g eines wasserfeuchten Raney-Nickels, 70% Nickel-Gehalt, entsprechend 4 Gew.-%, bezogen auf die Reaktionsmischung.

Die Kreisgasmenge beträgt 500 l/kg. Stunde, die Reaktionszeit bei 200°C 2 1/2 Stunden. Während dieser Zeit werden 32,2 l Wasserstoff dem Kreislauf zugeführt. Im Kreislauf erfolgt eine rasche Anreicherung an Ammoniak, daher wird ein Teil des Kreisgases im Nebenkreislauf durch verdünnte $H_2SO_4$ geführt, um im Kreisgas die Ammoniakkonzentration zwischen 35 und 50 Vol.-% zu halten. In der Schwefelsäure werden 0,102 Mol Ammoniak bestimmt, im Reaktionswasser (6,5 g) sind weitere 0,009 Mol Ammoniak enthalten. Nach dieser Reaktionszeit resultiert ein Gesamtamin in einer Aus beute von 97,9 Gew.-% mit einer Aminzahl von 19,64, das aus 7,2 Mol-% an primärem, 90,9 Mol-% an sekundärem und 1,9 Mol-% an tertiärem Amin besteht (Jodzahl 26). Anschliessend wird das Kreisgas eine weitere Stunde über verdünnte Schwefelsäure gefahren, wobei weitere 0,097 Mol Ammoniak erhalten werden, ferner werden während dieser Zeit weitere 4,2 l Wasserstoff eingeschleust, zusätzliches Reaktions-

wasser entsteht nicht. Nach dieser Zeit resultiert ein Amin mit einer Restjodzahl von 4 und einer Aminzahl von 18,31, das frei von primärem Amin ist und zu 97,6 Mol-% aus sekundärem Amin besteht, der Rest ist tertiäres Amin.

Beispiel 11

In der Kreislaufapparatur, beschrieben in Beispiel 1, werden vorgelegt:
50,0 g = 0,191 Mol Talgfettnitril (Jodzahl 55, Stickstoffgehalt nach Kjeldahl 5,35%),
150,0 g = 0,572 Mol hydrierter Talgfettalkohol (OH—Zahl 214) und
5,7 g wasserfeuchten Raney-Nickels mit einem Nickel-Gehalt von 79 Gew.-%, entsprechend einem Anteil an Raney-Nickel von 2 Gew.-%, bezogen auf die Reaktionsmischung.

Die Kreisgasmenge beträgt 500 l/kg. Stunde, die Reaktionstemperatur 200°C. Während der Reaktionszeit werden 10,5 l Wasserstoff kontinuierlich in den Kreislauf ingespeist, zusätzlich werden ab Erreichen einer Temperatur von 140°C über 1 1/4 Stunden aus einer Kältefalle 4,25 g = 0,241 Mol Ammoniak in den Kreislauf eingeführt. Während der Zeit der Ammoniakkeinspeisung liegt die Ammoniakkonzentration im Kreisgas zwischen 30 und 40 Vol.-%, sie sinkt dann bis zum Ende der Reaktionszeit nach 2 1/2 Stunden auf 8 Vol.-% ab. Nach dieser Zeit resultiert ein Gesamtamin in einer Ausbeut von 97,1 Gew.-% mit einer Jodzahl 10 und einer Aminzahl 19,07. Dieses Gesamtamin enthält 3,9 Mol-% primäres, 91,7 Mol-% sekundäres und 4,5 Mol-% tertiäres Amin. Das Kreisgas wird dann bei 200°C noch 1 Stunde lang weiter umgewälzt und dabei durch verdünnte Schwefelsäure geführt, um den Ammoniak zu entfernen. Während dieser Zeit werden weitere 1,4 l Wasserstoff zugeführt. Es resultiert schlieslich ein Amin mit einer Restjodzahl von 3 und einer Amin zahl von 18,92, das 1,3 Mol-% primäres, 93,3 Mol-% sekundäres und 5,4 Mol-% tertiäres Amin enthält.

Beispiel 12

In der Kreislaufapparatur, beschrieben in Beispiel 1, werden vorgelegt:
92,5 g = 0,355 Stearylnitril (Stickstoffgehalt nach Kjeldahl 5,34%),
107,5 g = 0,333 Mol Behenylalkohol (OH—Zahl 174), und 5,7 g wasserfeuchten Raney-Nickels Nickel-Gehalt 70%, entsprechend 2 Gew.-%, bezogen auf die Reaktionsmischung.

Es wird eine Kreisgasmenge von 500 l/kg. Stunde eingestellt und auf 200°C aufgeheizt, die Reaktionszeit beträgt 3,5 Stunden. Während des Aufheizens wird bei 140°C gasförmiger Ammoniak in einer Menge von 10% des Kreisgasvolumens in den Kreislauf geschleust. Während der Reaktionszeit werden 18 l Wasserstoff in den Kreislauf eingefahren. Die Ammoniakkonzentration beträgt bei Erreichen der Reaktionstemperatur 200°C 39 Vol.-%, nach 1/2 Stunde 28 Vol.-%, nach 1 Stunde 15 Vol.-% und am Ende der Reaktionszeit 3 Vol.-%. Es resultiert ein Gesamtamin in einer Ausbeute von 97,2 Gew.-% mit einer Aminzahl von 16,79, das aus 1 Mol-% an primärem, 94,3 Mol-% an sekundärem und 4,7 Mol-% an tertiärem Amin besteht.

Beispiel 13

In der Kreislaufapparatur, beschrieben in Beispiel 1, werden vorgelegt:
105,0 g = 0,401 Mol Talgfettnitril (Johzahl 55, Stickstoff-gehalt nach Kjeldahl 5,35%),
95,0 g = 0,362 Mol hydrierter Talgfettalkohol (OH—Zahl 212), und
17,2 g wasserfeuchtes Raney-Kobalt, Kobalt-Gehalt ca. 70 Gew.-%. entsprechend 6 Gew.-% Kobalt, bezogen auf die Reaktionsmischung.

Nach dem Füllen der Apparatur mit Wasserstoff wird ein Kreisgasumlauf von 500 l/kg. Stunde eingestellt und unter kräftigem Rühren auf 180°C aufgeheizt. Bei einer Temperatur von 140°C wird gasförmiger Ammoniak in den Kreislauf in einer Menge vom 10% des Kreisgasvolumens eingeschleust. Die Reaktionszeit beträgt 8 Stunden, während dieser Zeit werden 22,3 l Wasserstoff eingefahren. Unmittelbar nach Erreichen der Reaktionstemperatur beträgt die Ammoniakkonzentration im Kreisgas 13 Vol.-% nach 2,5 Stunden 35 vol.-%, nach 5 Stunden 21 Vol.-% und am Ende 5 Vol.-%. Es resultiert ein Gesamtamin in einer Ausbeute von 94,0% mit einer Jodzahl von 20 und einer Aminzahl von 17,97, das zu 11,9 Mol-% aus primärem, 86,4 Mol-% aus sekundärem und 1,8 Mol-% aus tertiärem Amin besteht.

Beispiel 14

In der Kreislaufapparatur, beschrieben in Beispiel 1, werden vorgelegt:
105,0 g = 0,401 Mol Talgfettnitril (Johzahl 55, Stickstoffgehalt nach Kjeldahl 5,35%),
95,0 g = 0,362 Mol hydrierter Talgfettalkohol (OH—Zahl 214), und
4 g eines Kupfer-Chromit-Katalysators, entsprechend 2 Gew.-%, bezogen auf das Reaktionsgemisch.

Kreisgasmenge 500 l/kg. Stunde, Reaktionstemperatur 250°C, Reaktionszeit 7 Stunden. Bei 140°C wird Ammoniak in einer Menge von 10% des Kreisgasvolumens zugegeben. Ferner werden während der Reaktionszeit 20,9 l Wasserstoff eingefahren. Die Ammoniakkonzentration steigt langsam an, sie beträgt nach 4,5 Stunden 25 Vol.-%, und geht dann langsam wieder zurück bis auf 13 Vol.-% am Reaktionsende. Es wird ein Gesamtamin mit 88,5 Gew.-% Ausbeute, einer Jodzahl von 27 und einer Aminzahl von 16,38 erhalten. Dieses besteht zu 10 Mol-% aus primärem, zu 82,3 Mol-% aus sekundärem und zu 7,7 Mol-% aus tertiärem Amin.

Beispiel 15

Entsprechend Beispiel 6 wird Octannitril und Tetraäthylenglykol-mono-n-butyläther in der Kreislaufapparatur 8 Stunden bei 200°C umgesetzt. Die Kreisgasmenge beträgt 500 l/kg. Stunde. Der Ammoniakspiegel im Kreisgas beträgt zu Beginn der Reaktion 465 Vol.-% und fällt auf 5 Vol.-% gegen Ende der Reaktion ab. Es werden 93,3 Gew.-% Gesamtamin mit einer Aminzahl von 24,85 erhalten. Davon sind 3,7 Mol-% primäres Amin, 80,5 Mol-% sekundäres Amin und 15,8 Mol-% tertiäres Amin.

147,4 g dieses Gesamtamins werden einer fraktionierten Destillation bei 9 mm Hg Druck unterworfen, wobei folgende Fraktionen erhalten werden:

| Kopftemperatur (°C) | Auswaage (g) |
|---|---|
| 70 bis 162 | 16,7 g |
| 162 bis 165 | 14,1 |
| 165 bis 232 | 17,6 |
| 232 bis 236 | 38,5 |
| 236 bia 275 | 19,9 |
| 275 bis 281 | 28,5 |
| Rückstand | 6,0 |
| Kältefalle | 1,8 |
| | 143,1 g |

Die bei einer Kopftemperatur von 232 bis 236°C übergehende Fraktion enthält gemäss gaschromatographischer Analyse 95 Gew.-% der Verbindung

$$C_8H_{17}—NH—(CH_2CH_2O)_4—n—C_4H_9$$

Die gaschromatographische Analyse wurde durchgeführt in einer 1,5 m hohen Säule von 2 mm Durchmesser, versehen mit einer Füllung von 5 Gew.-% "Silicon Fluid $QF_1$" auf "Chromosorg G—AW—DMCS" (zu beziehen durch die Firma Merck AG, Darmstadt); Trägergasströmung: 20 ml/min Stickstoff; Temperaturprogression: 80 bis 250°C mit 2°C pro Minute Steigung; Probenvolumen: 0,2 $\mu$l.

**Patentansprüche**

1. Verfahren zur Herstellung von sekundären aliphatischen Aminen aus aliphatischen Nitrilen und aliphatischen Alkoholen in flüssiger Phase unter Hindurchleiten von Wasserstoff in Gegenwart von Hydrier-Dehydrierkatalysatoren bei erhöhten Temperaturen und unter Abführung des Reaktionswassers, dadurch gekennzeichnet, dass man bei Temperaturen vom 120 bis 260°C unter praktisch drucklosen Bedingungen aliphatische Alkohole der Formel

$$R_1OH,$$

worin $R_1$ einen geradkettigen oder verzweigten, in $\alpha$-Stellung jedoch höchstens einfach verzweigten Alkyl-, Alenyl- oder mehrfach äthylenisch ungesättigten Kohlenwasserstoffrest mit 8—26 C—Atomen oder einen Alkoxyalkylrest der Formel $R_2(OX)_m$- mit einem Molekulargewicht von mindestens 130, in welcher $R_2$ ein geradkettiger oder verzweigter Alkyl-, Alkenyl- oder mehrfach äthylenisch ungesättigter Kohlenwasserstoffrest mit 1 bis 26 C—Atomen, X ein Rest

$$—CH_2CH_2—, —CH(CH_3)—CH_2—$$

oder $—CH_2—CH(CH_3)—$ und m eine ganze oder gebrochene Zahl zwischen 1 und 20 sein kann und innerhalb von m auch Kombinationen der Reste X vorliegen können, bedeutet,

oder Gemische solcher Alkohole mit aliphatischen Nitrilen der Formel

$$R_3—CN,$$

worin $R_3$ einen geradkettigen oder verzweigten Alkyl-, Alkenyl- oder mehrfach äthylenisch ungesättigten Kohlenwasserstoffrest mit 8 bis 26 C—Atomen oder den Rest eines Äthernitrils der Formel

$$R_4—(OX)_n—O(CH_2)_p$$

mit einem Molekulargewicht von mindestens 130, in welcher $R_4$ ein geradkettiger oder verzweigter Alkyl-, Alkenyl- oder mehrfach äthylenisch ungesättigter Kohlenwasserstoffrest mit 1 bis 26 C—Atomen, X ein Rest der obengenannten Bedeutung, n eine ganze oder gebrochene Zahl zwischen 1 und 20 oder null und p 1 oder 3 sein kann, wobei innerhalb von n auch Kombinationen der Reste X vorliegen können, bedeutet,

oder mit Gemischen solcher Nitrile in einem Molverhältnis Alkohol : Nitril von 90 : 10 bis 10 : 90 mit mindestens 2 Mol Wasserstoff pro Mol Nitril in innigen Kontakt bringt, wobei während der gesamten Reaktionszeit eine Ammoniakkonzentration zwischen 3 und 75 Vol.-% im Reaktionsgas aufrechterhalten wird.

2. Verfahren nach Anspruch 1, dadurch gekennzeichnet, dass während der gesamten Reaktionszeit eine Ammoniakkonzentration zwischen 3 und 60 Vol.-% im Reaktionsgas aufrechterhalten wird.

3. Verfahren nach Anspruch 1, dadurch gekennzeichnet, dass während der gesamten Reaktionszeit eine Ammoniakkonzentration zwischen 3 und 50 Vol.-% im Reaktionsgas aufrechterhalten wird.

4. Verfahren nach Anspruch 2, dadurch gekennzeichnet, dass das Molverhältnis Alkohol: Nitril von 70 : 30 bis 30 : 70 beträgt.

5. Verfahren nach Anspruch 2 und 3, dadurch gekennzeichnet, dass das Molverhältnis Alkohol : Nitril von 60 : 40 bis 40 : 60 beträgt.

6. Verfahren nach Anspruch 1 bis 5, dadurch gekennzeichnet, dass man das aus Wasserstoff und Ammoniak bestehende Reaktionsgas, das

gegebenenfalls zusätzlich 0 bis 50 Vol.-% Inertgas enthalten kann, im Kreislauf führt und das Reaktionswasser aus dem Kreislauf entfernt.

7. Verfahren nach Anspruch 6, dadurch gekennzeichnet, dass man im Reaktionsverlauf chargenweise oder kontinuierlich einen Teil des Kreisgases aus dem Kreislauf entfernt und durch frisches Reaktionsgas, gegebenenfalls unter Zusatz von Inertgas, ersetzt.

8. Verfahren nach Anspruch 1 bis 7, dadurch gekennzeichnet, dass der zur Aufrechterhaltung der Ammoniakkonzentration erforderliche Ammoniak mindestens teilweise von aussen zugefahren wird.

9. Verfahren nach Anspruch 1 bis 8, dadurch gekennzeichnet, dass Ausgangsalkohole und Ausgangsnitrile eingesetzt werden, die einen geradkettigen oder verzweigten Alkyl-, Alkenyl- oder mehrfach äthylenisch ungesättigten Kohlenwasserstoffrest mit 16 bis 22 C—Atomen besitzen.

10. Verfahren nach Anspruch 1 bis 8, dadurch gekennzeichnet, dass Alkohole mit dem genannten Aloxyalkylrest und Äthernitrile eingesetzt werden, in denen m = 1 bis 3 und n = 1 bis 3 ist.

11. Verfahren nach Anspruch 1 bis 10, dadurch gekennzeichnet, dass ein- oder mehrfach ungesättigte Alkohole und Nitrile eingesetzt werden und dass nach Beendigung der Bildung des sekundären Amins reiner Wasserstoff mit der flüssigen Phase in innigen Kontakt gebracht wied, gegebenenfalls unter Druckerhöhung auf etwa 4 bis 10 bar.

**Revendications**

1. Procédé de préparation d'amines secondaires aliphatiques à partir de nitriles aliphatiques et d'alcools aliphatiques en phase liquide, en présence de catalyseurs d'hydrogénation-déshydrogénation, à température élevée, avec passage d'un courant d'hydrogène et élimination de l'eau engendrée par la réaction, procédé caractérisé en ce qu'on met en contact intime, à des températures de 120 à 260°C, pratiquement sans pression, des alcools aliphatiques répondant à la formule

$$R_1OH$$

dans laquelle $R_1$ représente un radical alkyle, un radical alcényle ou un radical hydrocarboné à plusieurs doubles liaisons éthyléniques, chacun de ces radicaux contenant de 8 à 26 atomes de carbone et étant linéaire ou ramifié mais ayant, en position $\alpha$, au plus une ramification, ou représente un radical alcoxy-alkyle $R_2(OX)_m$—de poids moléculaire au moins égal à 130 dans lequel $R_2$ peut être un radical alkyle, un radical alcényle ou un radical hydrocarboné à plusieurs doubles liaisons éthyléniques, ce radical $R_2$ contenant de 1 à 26 atomes de carbone et étant linéaire ou ramifié, X paut être un radical $—CH_2CH_2—$ $—CH(CH_3)—CH_2—$ ou $—CH_2—CH(CH_3)—$ et m un nombre entier ou fractionnaire compris entre 1 et 20, et dans lequel l'enchaînement $—(OX)_m—$ peut renfermer des associations de radicaux X différents, ou des mélanges de tels alcools, et des nitriles aliphatiques répondant à la formule

$$R_3—CN,$$

dans laquelle $R_3$ représente un radical alkyle, un radical alcényle ou un radical hydrocarboné à plusieurs doubles liaisons éthyléniques, qui contient de 8 à 26 atomes de carbone et qui est linéaire ou ramifié, our représente le radical d'un éther nitrile $R_4—(OX)_n—O(CH_2)_p—$ de poids moléculaire au moins égal à 130 dans lequel $R_4$ peut être un radical akyle, un radical alcényle ou un radical hydrocarboné à plusieurs doubles liaisons éthyléniques, ce radical $R_4$ contenant de 1 à 26 atomes de carbone et étant linéaire ou ramifié, X un radical ayant la signification indiquée plus haut, n un nombre entier ou fractionnaire compris entre 1 et 20 ou égal à 0, et p le nombre 1 ou le nombre 3, et dans lequel l'enchaînement $—(OX)_n—$ peut renfermer des associations de radicaux X différents, ou des mélanges de nitriles de ce genre, dans un rapport molaire alcool-nitrile compris entre 90:10 et 10:90, avec au moins deux moles d'hydrogène par mole de nitrile, cela tout en maintenant, pendant toute la réaction, une concentration en ammoniac de 3 à 75% en volume dans le gaz réactionnel.

2. Procédé selon la revendication 1, caractérisé en ce qu'on maintient dans le gaz réactionnel, pendant toute la durée de la réaction, une concentration en ammoniac comprise entre 3 et 60% en volume.

3. Procédé selon la revendication 1, caractérisé en ce qu'on maintient dans le gaz réactionnel, pendant toute, la durée de la réaction, une concentration en ammoniac comrpise entre 3 et 50% en volume.

4. Procédé selon la revendication 2, caractérisé en ce que le rapport molaire de l'alcool au nitrile est compris entre 70 : 30 et 30 : 70.

5. Procédé selon l'une des revendications 2 et 3, caractérisé en ce que le rapport molaire de l'alcool au nitrile est compris entre 60 : 40 et 40 : 60.

6. Procédé selon l'une quelconque des revendications 1 à 5, caractérisé en ce que le gaz réactionnel, qui est constitué d'hydrogène et d'ammoniac et qui peut en outre contenir éventeullement de 0 à 50% en volume d'un gaz inerte, est conduit en circuit, et l'eau réactionnelle est éliminée du circuit.

7. Procédé selon la revendication 6, caractérisé en ce qu'on fait sortir du circuit au cours de la réaction, en discontinu ou en continu, une partie du gaz circulant et on la remplace par du gaz réactionnel frais, éventuellement additionné d'un gaz inerte.

8. Procédé selon l'une quelconque des revendications 1 à 7, caractérisé en ce que l'ammoniac nécessaire pour maintenir la concentration en ammoniac est introduit, au moins partiellement, de l'extérieur.

9. Procédé selon l'une quelconque des revendications 1 à 8, caractérisé en ce qu'on se sert d'alcools et de nitriles de départ qui contiennent un radical alkyle, un radical alcényle ou un radical hydrocarboné à plusieurs insaturations éthyléniques, chacun de ces radicaux pouvant être linéaire ou ramifié et contenant de 16 à 22 atomes de carbone.

10. Procédé selon l'une quelconque des revendications 1 à 8, caractérisé en ce qu'on utilise des alcools renfermant un radical alcoxy-alkyle tel que défini dans lesquels m est un nombre de 1 à 3, et des éther-nitriles dans lesquels n est un nombre de 1 à 3.

11. Procédé selon l'une quelconque des revendications 1 à 10, caractérisé en ce qu'on utilise des alcools et des nitriles mono-insaturés ou polyinsaturés, et en ce qu'après la fin de la formation de l'amine secondaire on met de l'hydrogène pur en contact intime avec la phase liquide, éventuellement en élevant la pression à environ 4 à 10 bars.

**Claims**

1. Process for the manufacture of secondary aliphatic amines from aliphatic nitriles and aliphatic alcohols in the liquid phase by passing hydrogen through the reaction mixture in the presence of hydrogenation/dehydrogenation catalysts at elevated temperatures and with removal of the water of reaction, which is characterized by bringing aliphatic alcohols of the formula

$$R_1OH$$

in which $R_1$ denotes a straight-chain or branched, but in the $\alpha$-position at most singly branched, alkyl, alkenyl or ethylenically multi-unsaturated hydrocarbon radical having 8 to 26 C atoms or an alkoxyalkyl radical of the formula $R_2(OX)_m$— with a molecular weight of at least 130, in which $R_2$ is a straight-chain or branched alkyl, alkenyl or ethylenically multi-unsaturated hydrocarbon radical having 1 to 26 C atoms, X is a radical $-CH_2CH_2-$, $-CH(CH_3)-CH_2-$ or $-CH_2-CH(CH_3)-$ and m is an integer or fraction between 1 and 20 and, within m, combinations of the radicals X can be present, or mixtures of such alcohols into intimate contact, at temperatures of 120 to 260°C and under conditions of virtually atmospheric pressure, with aliphatic nitriles of the formula

$$R_3-CN$$

in which $R_3$ denotes a straight-chain or branched alkyl, alkenyl or ethylenically multi-unsaturated hydrocarbon radical having 8 to 26 C atoms or a radical of an ether-nitrile of the formula $R_4-(OX)_n-O(CH_2)_p-$ with a molecular weight of at least 130, in which $R_4$ is a straight-chain or branched alkyl, alkenyl or ethylenically multi-unsaturated hydrocarbon radical having 1 to 26 C atoms, X is a radical as defined above, n is an integer or fraction between 1 and 20, or zero, and p is 1 or 3 and, within n, combinations of the radicals X can also be present, or with mixtures of such nitriles, in a molar ratio of alcohol : nitrile of 90 : 10 to 10 : 90, and with at least 2 moles of hydrogen per mole of nitrile, whereby maintaining an ammonia concentration of between 3 and 75% by volume in the reaction gas during the entire reaction time.

2. A process according to claim 1, characterized by maintaining an ammonia concentration of between 3 and 60% by volume in the reaction gas during the entire reaction time.

3. A process according to claim 1, characterized by maintaining an ammonia concentration of between 3 and 50% by volume in the reaction gas during the entire reaction time.

4. A process according to claim 2, wherein the molar ratio of alcohol : nitrile is 70 : 30 and 30 : 70.

5. A process according to claims 2 and 3, wherein the molar ratio of alcohol : nitrile is 60 : 40 to 40 : 60.

6. A process according to claims 1 to 5, characterized by cycling the reaction gas, which consists of hydrogen and ammonia and optionally can additionally contain 0 to 50% by volume of inert gas, and removing the water of reaction from the cycle.

7. A process according to claim 6,which is characterized by removing, in the course of the reaction, part of the circulating gas batchwise or continuously from the cycle and replacing it with fresh reaction gas, optionally with the addition of inert gas.

8. A process according to claims 1 to 7, which is characterized by supplying at least part of the ammonia required to maintain the ammonia concentration from outside into the cycle.

9. A process according to claims 1 to 8, which is characterized by using starting alcohols and starting nitriles having a straight-chain or branched alkyl, alkenyl or ethylenically multi-unsaturated hydrocarbon radical having 16 to 22 C atoms.

10. A process according to claims 1 to 8, which is characterized by using alcohols containing the said alkoxyalkyl radical, and ether-nitriles, in which m is 1 to 3 and n is 1 to 3.

11. A process according to claims 1 to 10, which is characterized by using mono- or multi-unsaturated alcohols and nitriles and, after the formation of the secondary amine is completed, bringing pure hydrogen into intimate contact with the liquid phase, the pressure optionally being increased to about 4 to 10 bars.